# EUROPEAN PATENT APPLICATION

(11) **EP 1 679 035 A1**
(43) Date of publication of application: **12.07.2006**
(21) Application number: 06290011.3
(22) Date of filing: 04.01.2006
(51) Int. Cl.: A61B 5/06, A61B 8/06, A61B 5/026

(54) **Device for analysis and measurement of images and medical signals**

(30) Priority: 06.01.2005 FR 0500111
(71) Applicant: Darlas, Yves, 41000 Blois (FR)
(72) Inventor: Darlas, Yves, 41000 Blois (FR)
(74) Representative: Burtin, Jean-François

(57) **Abstract**

The present invention relates to the field of the essentials of life and more particularly, to the field of biological analysis.

More particularly it relates to a device for analysis, and detection for the detection of disturbances of blood flow in the different regions of the organism, in patients capable of demonstrating alterations in blood flow in different organs of the body, in particular in coronary flow, which includes the measure of the blood flow before and after a physical, medicinal and/or neurosensory stimulation test, which modifies the blood flow in the region considered by means of a tracer the fixation of which is measured, and its elimination rate is determined with respect to a control value using calculation software, during current medical imaging investigations, without any supplementary constraint or adverse effect for the patients.

Use for testing cerebral, cardiac or tumorous vascularization disturbances.

## Description

The present invention relates to the field of the essentials of life and, more particularly, to the field of measuring certain biological parameters.

More particularly it relates to a diagnosis and prognosis device for analysis, and detection of medical images and signals relating to diagnosis and prognosis of disturbances of circulatory flow in the different regions of the body, in patients capable of demonstrating alterations in blood flow in different organs of the body, and in particular in coronary flow, during current medical imaging investigations, without any supplementary constraint or a adverse effect for the patients.

Specifically it relates to a device for measuring and analysing blood flow during a normal examination, with or without the addition of an agent which increases or modifies blood flow, which consists of determining the blood flow before and after a physical, medicinal and/or neurosensory stimulation test, which modifies the blood flow in a defined region, using a tracer the fixation of which is measured, so that its fixation and/or elimination rate are determined with respect to a control value using calculation software.

The device for measuring and analysing the results according to the invention has the great advantage of being able to detect disturbances in the blood flow in different organs of the body, such as for example the brain or the heart, as well as in tumorous cells. It is therefore possible to detect abnormalities well before pathological symptoms are noticed and therefore the doctors be able to take the therapeutic or surgical measures which are required, as a preventive or prophylactic means.

More precisely, as regards coronary flow, the rate at which the tracer is fixed on the cardiac apparatus and the effects of exertion test on coronary flow are determined. This procedure involves no additional operation on the patient. The device only comprises, but not limitatively, a means for the collection of digital data, their processing and their analysis so as to establish a diagnostic, prognostic or therapeutic evaluation of the functioning of the organs considered, and in particular of the heart.

In this way, the risk of accidents is avoided, in particular at the cardiac level, linked to exertion tests and the consequences of which, in certain cases, prove to be dramatic. To this end, an utilization, of a pharmacodynamic agent is carried out which modifies the blood flow, either by increasing it, or by reducing it and the variations in the blood flow are measured.

A pharmacological agent which increases the blood flow is, for example, dipyridamole, adenosine or dobutamine.

A pharmacological agent which reduces the blood flow is, for example, atropine or its derivatives.

Thus, the administration of a pharmacological agent which increases the blood flow has the effect of increasing the flow in the arteries, and this increase, in particular as regards heart beat, manifests itself as during an exertion test, but this effect is rapid and transient, such that is can be easily controlled.

The simultaneous or consecutive utilization of a tracer allows the development and concentration of such a product to be monitored during the test. The tracer must essentially have a determination ability by fixing on the tissues and by allowing the evaluation of its fixation, elimination or circulation rate. The fixation of the tracer can occur according to a so-called cellular or extracellular "capillary compartment" model.

In fact, it is known that the vascular system, and above all the cardiac apparatus, constitutes a closed system, the tissue distribution kinetics of which for a given time, such as the tissue distribution time, can be assimilated into a closed compartmental kinetic model, arranged in the circulatory flow.

The tracer used in the method according to the invention is constituted by a gamma ray emitter, and in particular a positon emitter.

The tracer can also be a fluorescent product or a product capable of exhibiting fluorescence, such as for example eosine, fluorescein, erythrosine.

The tracer can also be a contrast product, such as for example an iodinated product, such as for example Telebrix, Telebrix meflumine or Iopamiron.

The contrast product used as a tracer is advantageously a contrast product which is visible by medical imaging (MRI), such as for example a derivative of gadolinium.

The contrast product used as a tracer can also be a product which is detectable by ultrasonography or by ultrasonic Doppler effect, such as for example Ecovist or Levovist.

After administration, the fixation of the tracer in the region concerned is determined, depending on the case, using an X-ray imaging device, such as for example a gamma camera, an X-ray scanner, a positon emission tomography camera, or any MRI imaging device.

According to the invention, the tracer is preferably a radiopharmaceutical or a positon emitter or a thallium isotope tracer. A radiopharmaceutical tracer can also be advantageously used chosen from the radioactive isotopes of technetium, and in particular metastable technetium Tc⁹⁹, the radioactive isotopes of indium and the radioactive isotopes of iodine.

Preferably, a radioactive tracer labelled with metastable Technetium Tc⁹⁹, such as Tetrofosmine or the product called MIBI (Dupont Pharma) or MYOWIEW (G.E) is used as tracer. Measurement of the fixation and measurement of the circulatory flow of the tracer allows an elimination rate to be calculated with respect to a control value constituted by the parameters of the patient, determined under the same conditions, without administration of a pharmacological agent increasing or reducing the circulatory flow. This measurement is carried out using a calculation software, such as for example that filed by the Applicant at the Program Protection Agency in Paris (France) under the number IDDN FR 001-1501011-000-SP2004/00 and called "CORYFLOW".

The method according to the invention has a particularly valuable use for the determination or the detection of early cardiac disturbances, well before the appearance of any pathological phenomena, such as for example in diabetic subjects who present a significant risk factor.

The method according to the invention also has a significant use for studying vascularization phenomena, in particular for studying cerebral blood circulation, either after a cerebral vascular accident, or earlier when a hypertensive or atherosclerotic patient can present symptoms of a reduction in the blood perfusion of the brain.

Moreover, the method according to the invention has a significant use for studying the vascularization of tumorous tissues. It is known, in fact, that the tumorous tissues are frequently congested as a result of the anarchic development of a neovascularization. Any measure which leads to a reduction of the vascularization in such an area can lead to an absence of perfusion in this region, and therefore, a reduction in the size of the tumorous tissue.

The method according to the invention will be defined before precisely on the basis of the operating method detailed below.

The same analysis method can also be of use for the determination or the quantification of other biological parameters. The attached flow diagram provides an explanation of the use of the calculation software.

The same analysis method can also be applied to the determination of the perfusion of other tissues, by the use of appropriate tracers.

### OPERATING METHOD

- Insert the CD-ROM into the computer,
- Leave to spin for a short while
- Then open "My Computer"
- Click on MID
- Then go to the name of a patient (for example called Mr. X)
- With the mouse, right- click on "send to..." then "My documents"
- Then remove the CD-ROM
- Then go to "My documents"
- Go to "Mr. X"
- Use "copy" then "paste" a good ten times (or twenty times, or more) in order to obtain as many copies as there are patients to treat
- **IMPORTANT NOTE** : **Only work on the copies, as the file "Mr. X" is the template and all that is required is to copy it.**
- The preliminary work consists of naming the files with the name of the patient to be treated. For example, in the case of a patient called "Mrs Cunegonde DUPONT".
- Select one of the copies of the file "Mr. X", rename this copy "cunegonde dupont 01", open the file : there are three EXCEL files inside ; rename them as "repos calcul cunegonde dupont 01" ("cunegonde dupont 01 rest calculation"), "calcul effort cunegonde dupont 01" ("cunegonde dupont 01 exertion calculation"), "resultats cunegonde dupont 01" ("cunegonde dupont 01 results")
- The assignment "repos" ("rest") or "effort" ("exertion") must be complied with absolutely as it is presented ; above all do not change it...
- Then open the file "repos calcul cunegonde dupont 01" ("cunegonde dupont 01 rest calculation"), then go to the raw data table ( everything will now happen here) and, in the order :
   1. enter the raw data : columns B, C, D and F, G, H
   2. go to the graph "bolus bruité choix des bornes" ("bolus triggering a signal choice of limits")
   3. visually select by eye "temps de début de calcul" ("start time of bolus") and above all "temps de fin du bolus" ("finish time of bolus") then the time of "fin du calcul" ("end calculation") ;
   4. go to the raw data and carefully enter these four times in column, also enter the dimensions of the ROIₛ.
   5. observe the various graphs which show the result,
   6. For the late phase, go to "courbes tardives" ("late graphs") to select the time ; do not forget the dimensions of the ROIs, and the time lapsed between the early injection and the acquisition in the late phase
   7. Once this is finished, record and open the file called "effort calcul cunegonde dupont 01" ("cunegonde Dupont 01 exertion calculation"),
   8. then repeat the same operations
   9. then, all that remains is to open the file "resultats cunegonde dupont 01" ("cunegonde dupont 01 results")
   10. It is important to note that if, for the same patient, one wishes to carry out another vascular and/or cardiac ROI, everything must be done as if for another patient called "cunegonde dupont 02".

The attached graphs and Table I demonstrate the digital values obtained for a patient by using the device according to the invention in a test which determines the coronary flow reserve (CFR %) after administration of dipyridamole and by using a tracer based on Technetium Tc₉₉m.

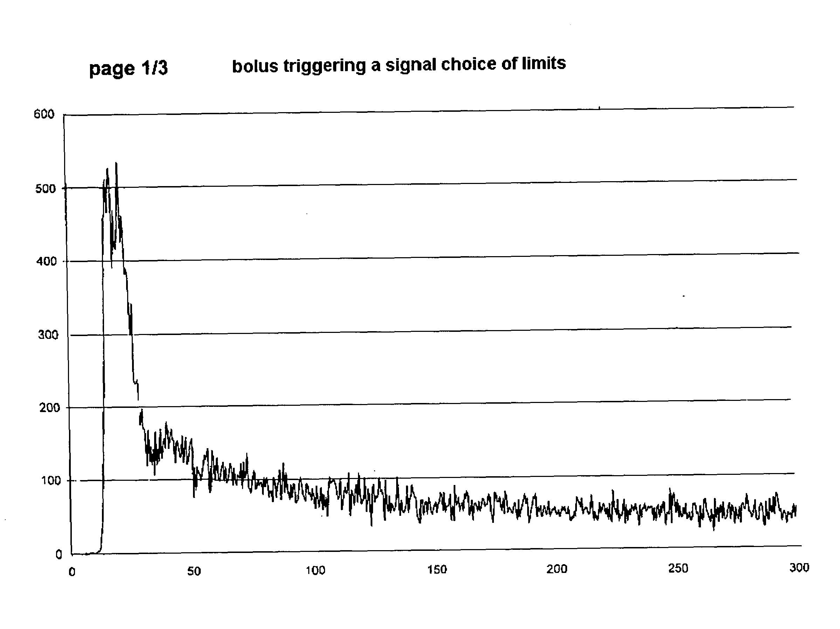

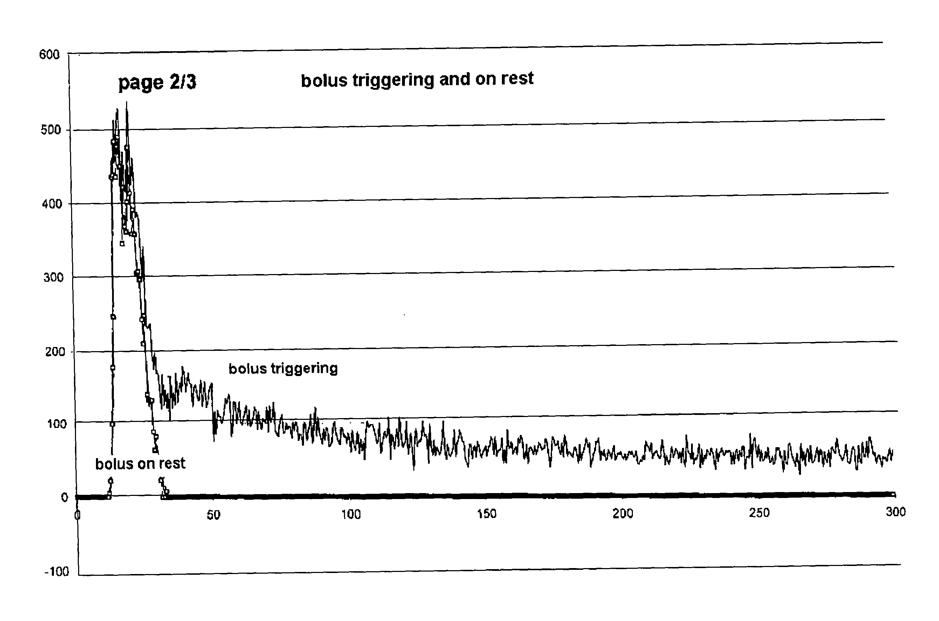

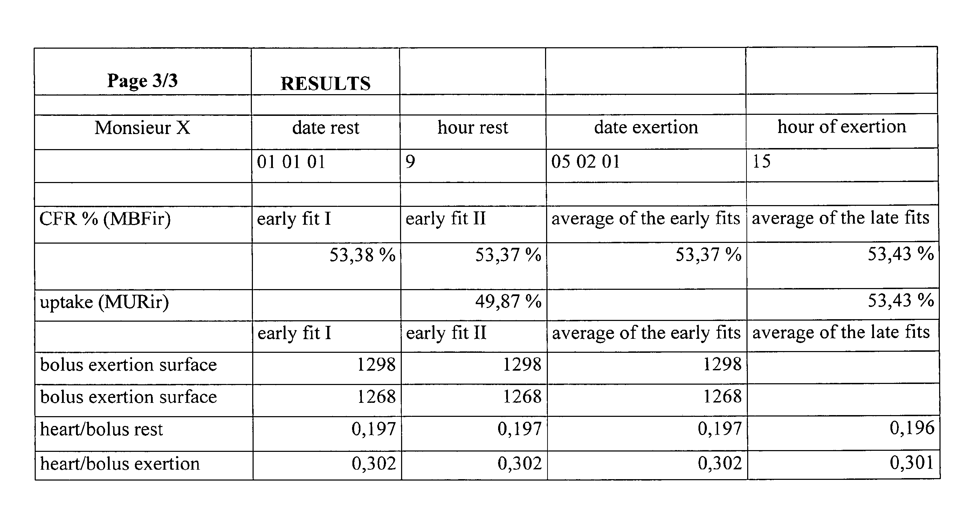

## Claims

1. A device for the analysis and detection of medical signals and images for the prognosis of disturbances of circulatory flow in the different regions of the organism which consists in a means for measuring the blood flow before and after a physical, medicinal and/or neurosensory stimulation test, which modifies the blood flow, using a tracer, the fixation and circulation of which is measured, and its fixation and elimination rates are determined with respect to a control value using a calculation software, during current medical imaging investigations, without any supplementary constraint or adverse effect for the patients.

2. Device for the analysis, detection and/or prognosis of disturbances of the blood flow according to claim 1, the target parameter being the coronary blood flow, which consists in measuring said flow before and after utilization of a product which modifies the coronary flow or after an exertion, by means of a tracer, the measurement of the fixation and the circulation of which is measured for determining the tissue fixation rate with respect to a control value, using calculation software.

3. Analysis and detection device according to claim 1 or claim 2,
in which
the tracer is a gamma ray emitter.

4. Analysis and detection device according to claim 1 or claim 2,
in which
the tracer is a gamma positon emitter.

5. Analysis and detection device according to claim 1,
in which
the tracer is a fluorescent product.

6. Analysis and detection device according to claim 1 or claim 2,
in which
the tracer is a contrast product.

7. Analysis and detection device according to claim 6,
in which
the contrast product is an iodinated product.

8. Analysis and detection device according to claim 1 or claim 2,
in which
the tracer is a contrast product which is visible by MRI.

9. Analysis and detection device according to claim 8,
in which
the contrast product which is visible by MRI, is a derivative of gadolinium.

10. Analysis and detection device according to claim 1 or claim 2,
in which
the contrast product is a product which is detectable by ultrasonography or by ultrasonic Doppler effect.

11. Analysis and detection device according to claim 1 or claim 2,
in which
the fixation of the tracer is measured using an X-ray scanners, positon emission tomography cameras, or an MRI imaging device.

12. Analysis and detection device according to claim 1 or claim 2,
in which
the product which modifies the coronary flow is selected among the compounds which increase the coronary flow and the products which reduce the coronary flow.

13. Analysis and detection device according to claim 12,
in which
the product which increases the coronary flow is selected among pyridamole, adenosine and dobutamine.

14. Analysis and detection device according to claim 12,
in which
the product which reduces the coronary flow is atropine or its derivatives.

15. Analysis and detection device according to claim 1 or claim 2,
in which
the tracer is a radio-pharmaceutical tracer, a positon emitter or an isotope of thallium.

16. Analysis and detection device according to claim 15,
in which
the radio-pharmaceutical tracer is selected among the derivatives of technetium Tc⁹⁹, the radioactive isotopes of indium and the radioactive isotopes of iodine.

17. Analysis and detection device according to claim 15 or claim 16,
in which
the radioactive tracer is a tracer labelled with metastable technetium Tc⁹⁹, called tetrofosmine.

18. Analysis and detection device according to one of the preceding claims,
in which
the calculation software is that filed at the Program Protection Agency under the number IDDN FR 001-150011-000-SP2004/00 and called "CORYFLOW"..

19. Analysis and detection device according to claim 1 or claim 2
in which
the control value is namely that of the coronary flow reserve, before or after an exertion test or pharmacological treatment.

20. Analysis and detection device according to claim 1
measures which
the blood flow in the tumorous tissues, before and after use of a radio-pharmaceutical tracer.

21. Analysis and detection device according to claim 1,
which utilizes
the calculation software defined under the denomination "CORYFLOW" with a view of studying organic disorders linked to the vascularization of the brain, heart and/or tumorous tissues.
